# EUROPEAN PATENT APPLICATION

(11) **EP 1 674 060 A1**
(43) Date of publication of application: **28.06.2006**
(21) Application number: 04030647.4
(22) Date of filing: 23.12.2004
(51) Int. Cl.: A61F 11/08

(54) **Method for providing a user with a hearing protection earplug**

(71) Applicant: PHONAK AG, 8712 Stäfa (CH)
(72) Inventor: Haussmann, Mathias, 8005 Zürich (CH)
(74) Representative: Schwan - Schwan - Schorer

(57) **Abstract**

The invention relates to a method for providing a user with a hearing protection earplug including a shell to be worn at least in part in the ear canal of the user, comprising: (1) collecting configuration data about audio needs of the user; (2) collecting data of the inner shape of the outer ear and ear canal of the user by measuring the actual inner shape of the user's outer ear and ear canal; (3) determining, based on said configuration data and said actual ear shape data, the desired shape of the shell; (4) manufacturing said shell based on said determined desired shape by an additive layer-by-layer build-up process; (5) producing a hearing protection earplug from said shell; (6) fitting said hearing protection earplug to the user for verifying that said hearing protection earplug complies with the configuration data collected in step (1), wherein at least one of steps (1), (2), and (6) is carried out at a transportation hub, a in a mobile location or at a site of the company for which the user works.

## Description

The invention relates to a method for providing a user with a hearing protection earplug including a customized shell, i.e. a shell having an outer surface individually shaped according to the inner shape of the user's outer ear and ear canal.

US 6,731,997 B2 relates to a process for manufacturing a hearing device, such as a noise protection device, comprising a customized shell, wherein first an individual data set is gathered for each user, comprising information regarding the individual user's audio needs such as hearing loss data, information regarding the three-dimensional shape of the user's outer ear and ear canal, and information regarding the individual user's non-audio needs. The individual user's data set is input into a computer system comprising a local computer and a remote computer via an internet-kind network or via a data carrier such as a CD or tape. 3D shape data of the user's outer ear and ear canal is obtained by taking an impression of the ear, which then undergoes scanning; alternatively, the user's ear is directly scanned. The impression taking and the scanning processes may be carried out at the location of a consultant who may be a retailer. Based on the gathered user's data set a hearing device adapted to the individual user is conceived and visualized at the consultant's place for being approved by the user and the consultant. Once the hearing device has been approved, the corresponding manufacturing data may be used in an assembling process wherein an individually shaped customized shell is produced and is combined with standard hardware elements, such as microphones, signal processing units, loudspeakers etc. Such hardware manufacturing may be carried out at the consultant's place and occurs just in time. The functional hearing device or a dummy thereof is supplied to the user at the consultant's place for final approval or for collecting fitting data if approval of the device or the dummy is denied. The customized shell is produced by an additive layer-by-layer build-up process. Such processes are also known as rapid prototyping. In case an already existing hearing device is to be updated or repaired or to be newly adapted, the manufacturing process may be carried out based on the individual user's data set collected for the already existing hearing device and stored in a database.

Examples for the use of additive layer-by-layer build-up processes for the customized shell of a hearing device are given, for example, in US 2003/0133583 A1.

It is a first object of the invention to provide for a method for providing a user with a hearing protection earplug, wherein the method is particularly convenient for the user.

It is a second object of the invention to provide for such a method which is particularly fast.

It is a third object of the invention to provide for such a method which allows for a particularly efficient adaptation of a hearing protection earplug to the individual audio needs of a user.

These objects are achieved by methods for providing a user with a hearing protection earplug as defined in claims 1, 5, 10, 12, and 15, respectively.

The methods according to claims 1, 5 and 12 are beneficial in that they are particularly convenient for the user, since at least one of the steps in which the user is personally involved in the production process, i.e. the steps of collecting configuration data about the user's audio needs, collecting data of the inner shape of the user's outer ear and ear canal by measuring the actual inner shape of the user's outer ear and ear canal, and fitting the hearing protection earplug to the user for verifying that the hearing protection earplug complies with the collected configuration data, is carried out at a location which is easily accessible by the user, namely a transportation hub, a mobile location, or a site at the company for which the user works.

The methods as defined in claims 10 and 15, respectively, are particularly fast, since fitting data regarding the hearing protection earplug is collected, and is permanently stored together with the ear shape data and the configuration data explored in order to manufacture the shell of a second earplug, or since at least one of the actual earplug production steps is carried out in a time zone different to the time zone in which the data collection and fitting steps are carried out.

The process basic to all embodiments of the invention includes the following basic steps:
(1) Collecting configuration data about the user's audio needs;
(2) collecting data of the ear shape of the user's outer ear and ear canal by measuring the actual inner shape of the users outer ear and ear canal;
(3) determining, based on the configuration data and the actual ear shape data, the desired shape of the shell of the hearing protection earplug to be produced, which is to be worn at least in part in the user's ear canal;
(4) manufacturing the shell based on the determined desired shape by an additive layer-by-layer build-up process;
(5) producing the hearing protection earplug from the shell; and
(6) fitting the hearing protection earplug to the user for verifying that the hearing protection earplug complies with the configuration data collected in step (1).

According to one aspect of the invention, at least one of steps (1), (2) and (6) is carried out at a transportation hub, such as an airport or a central station of public transportation, for example, a central train station, a central subway station or a central tramway station. According to this aspect of the invention, preferably at least steps (2) and (6) are carried out at the transportation hub and most preferably all steps (1) to (6) are carried out at the transportation hub.

According to another aspect of the invention at least one of steps (1), (2) and (6) is carried out in a mobile location, such as a bus, a truck or a tent. According to this aspect, preferably at least steps (2) and (6) are carried out in the mobile location and most preferably all of steps (2) to (6) are carried out at the mobile location.

According to a further aspect of the invention at least one of steps (1), (2) and (6) is carried out at a site at the company for which the user works. According to this aspect, preferably at least steps (2) and (6) are carried out at a site at the company for which the user works and most preferably all of steps (1) to (6) are carried out at a site of the company for which the user works.

According to a still further aspect of the invention, at least one of steps (3) to (5) is carried out in a time zone different to the time zone in which steps (2) and (6) are carried out. According to this aspect, preferably steps (3) and (4) are carried out in different time zones.

By distributing the process on different time zones the overall process can be accelerated since the availability of manpower is extended, in the extreme to a 24 hours basis. For example, modeling and manufacturing of the shell and production of the hearing protection earplug from the shell could occur overnight, i.e. during day time in the time zone in which the user lives.

Carrying out at least one of steps (1), (2) and (6) at a transportation hub, a mobile location or at a site of the company for which the user works is particularly convenient for the user, since he will already visit such locations for other reasons.

According to another aspect of the invention, in step (6) data regarding the fitted hearing protection earplug, such as the mechanical acoustic attenuation achieved, is collected and said configuration data, said ear shape data and said fitting data are permanently stored and are used for manufacturing a second shell and a second earplug. Thereby a particularly fast process for producing a second earplug is achieved, since all data necessary for producing the second earplug is already available from the manufacturing process of the previous, i.e. first, earplug so that no further data collecting is necessary.

According to this aspect, subsequent to step (6) an order including modified configuration data for said second earplug may be collected from the user or the company for which the user works, and said second earplug may be produced based on modified configuration data, said ear shape data, and said fitting data. Thereby modified hearing protection earplugs can be produced in a simple and efficient manner in order to adapt the hearing protection earplugs to changes in the audio needs of the user, for example, if the user is exposed to a different noise environment, such as a new machine, or if the user's hearing loss has changed, or if his communication needs in the noise environment have changed, etc. Preferably the modified configuration data is collected on-line.

Preferably the method according to this aspect forms part of a service package, in which a company, whose workers are to be provided with hearing protection earplugs, does not buy the individual hearing protection earplugs from the supplier but rather buys a service package which includes the provision of identical or modified hearing protection earplugs for replacing the original hearing protection earplugs in case of damage or loss of the hearing protection earplugs or in case of changes of the worker's audio needs.

Further, by storing the monitored data of the achieved hearing protection, i.e. the actual acoustic attenuation achieved by the earplugs, it is possible, for example, to avoid liability problems of the company which might occur if a worker of the company suffers a hearing loss.

By storing all data collected for the previous, i.e. first, hearing protection earplug, on-line ordering of an identical or modified second earplug is enabled.

The following optional features relate to all aspects of the invention.

Preferably step (2) is carried out according to a standardized process in order to achieve reliable and reproducible ear shape data.

Preferably, steps (4) and (5) are carried out at the same site, while step (3) maybe carried out at a site different from the site at which steps (4) and (5) are carried out. In step (2), the ear shape data maybe collected by taking an impression of the inner shape of the user's outer ear and ear canal and scanning the impression or by direct in-vivo scanning of the inner shape of the user's outer ear and ear canal. Scanning is preferably carried out optically, for example, by using a laser. While in general the impressing taking and the impression scanning could occur at different locations, it is preferred to scan the impression at the same location where it has been taken in order to avoid shipping of the impression. The collected ear shape data may be transferred via the internet to the site where step (3) is carried out. In addition, the user may be provided with a personal copy of a digital data set representative of the measured ear shape for personal use by the user. Such personal copy of the ear shape data may be used for producing a second, i.e. further, earplug, i.e. by a manufacturer different from the manufacturer of the original earplug without the need for again collecting the ear shape data by measurements.

The configuration data collected in step (1) may include data regarding the manner of use of the hearing protection earplug as intended by the user, data regarding a hearing disability of the user and data regarding the expected noise exposure in the environments in which the earplug is intended to be worn. The data regarding the manner of intended use of the earplug may include the communication needs of the user when wearing the earplug, in particular the need for communication with other workers. For example, if speech communication with other workers is necessary, the earplug would have to be designed as an active hearing protection device including a microphone, an audio signal processing unit and a speaker in order to selectively transmit speech signals to the user's ear when wearing the earplug in noisy environments. The configuration data also may include data regarding regulations of the allowed noise exposure of workers.

In step (3), functional components of the earplug may be selected from a library of functional components, with the selection being based on the configuration data and the ear shape data. Such functional components may include external components such as microphones, speakers, batteries, signal processing circuitry, radio frequency transmitters/receivers, etc., and components which are integrated within the shell, i.e. components which are created by corresponding shaping of parts of the shell, such as passive acoustic filters (realized as, for example, resonance cavities within the shell), acoustic valves which may be operated by the user or automatically by excessive sound pressure levels in order to intentionally or automatically change the acoustic attenuation provided by the earplug, etc. Thereby the earplug can be individually adapted to the needs of the user and the desired functions of the earplug. Consequently, the outer shape of the shell is primarily determined by the ear shape data in order to achieve optimized retention and wearing comfort of the earplug within the user's ear canal, while the internal structure of the shell is primarily determined by the selected functional components, either by forming receptacles for receiving external electronic units or by shaping the interior of the shell in the desired functional manner.

If the functional components are external components, they are assembled in step (5) with the shell. If necessary, such functional components are adjusted in step (6) for achieving the desired functionality.

Preferably, in step (3) data representative of the determined desired shape is permanently stored in order to have it readily available if, for example, the earplug is to be replaced by an identical new one in case of loss or damage of the original earplug.

In step (4) the shell is preferably manufactured by layer-by-layer laser sintering of a powder material, preferably polyamide powder, or by laser stereo-lithography or photopolymerization. An overview regarding such additive layer-by-layer build-up processes for manufacturing customized shells if hearing devices can be found, for example, in US 2003/0133583 A1 or US 6,533,062 B1. Preferably the shell is a hard shell having an elasticity from shore D85 to D65.

Preferably, step (6) includes at least one of the following sub-steps: in-situ measuring the acoustic attenuation achieved by the earplug when worn by the user, providing the user with handling instructions, visible check of proper fit of the shell with the user's ear canal, and adjusting acoustical and mechanical properties of the earplug according to the desired functionality. Further, as already mentioned above, in step (6) data obtained during the fitting step, such as data regarding the actually achieved acoustic attenuation of the earplug, may be stored for further use when producing a further earplug for the same user or for documentation purposes.

Examples of invention will be illustrated by reference to the attached drawings.
- Fig. 1: shows a schematic block diagram of an example of a method according to the invention; and
- Fig. 2: shows a schematic block diagram of another example of a method according to the invention.

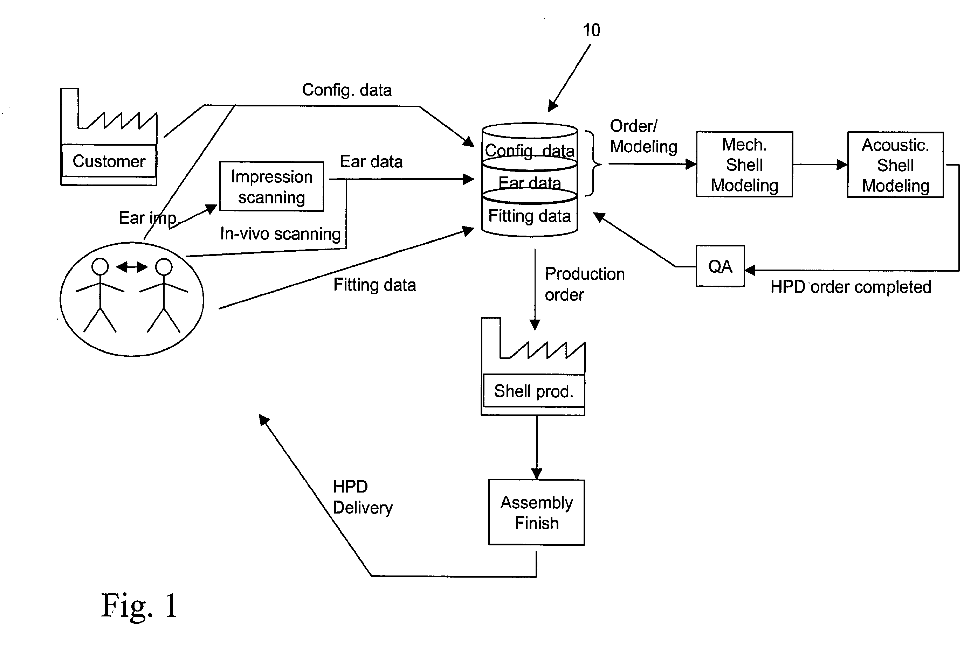

Fig. 1 shows an example of a method for providing a user with a hearing protection earplug, wherein the user is a worker of a company who is exposed regularly or from time to time to noisy working environments. According to this process, configuration data about the user's audio needs, such as the manner of the intended use of the hearing protection earplug, data regarding a hearing disability of the user and the expected noise exposure of the user are collected from the company for which the user works or directly form the user.

In addition, data of the inner shape of the user's outer ear and ear canal are collected by measuring the actual inner shape of the user's outer ear and ear canal. This may be achieved by taking an impression of the user's ear which is subsequently scanned or by direct in-vivo scanning of the user's ear.

The collected configuration data and ear shape data are entered into a data base 10 which is accessible by the manufacturer of the hearing protection earplug. Based on the configuration data and the ear shape data the desired shape of the shell of the hearing protection earplug is determined by mechanical and acoustic shell modelling. In this modelling step additional functional components of the earplug may be selected from a corresponding library in order to achieve the desired functionality of the earplug determined by the collected configuration data. The finally modelled shell then undergoes a quality assurance step and data representative of the modelled (i.e. desired) shape of the shell is stored in the database 10 together with data regarding the added functional components.

Upon a corresponding production order the shell is manufactured by an additive layer-by-layer build-up process based on the modelled shell shape data stored in the database 10. The shell then is assembled with the selected additional functional components and may undergo further finishing steps in order to complete production of the hearing protection earplug.

The product, i.e. a pair of hearing protection earplugs, then is delivered to the user for fitting the hearing protection earplugs to the user in order to verify that the hearing protection earplugs comply with the configuration data initially collected. By this fitting procedure it is ensured that the initially desired functionality of the earplugs has actually been achieved. The fitting procedure may include in-situ measuring of the acoustic attenuation of the earplug when worn by the user, providing the user with handling instructions, visible check of proper fit of the shell within the user's ear canal and adjusting acoustical and mechanical properties of the earplug, for example, by adjusting the settings of an active electro-acoustic unit, (i.e. a unit consisting of a microphone, an audio signal processing and a speaker) assembled with the shell.

Data obtained by this fitting procedure, such as the actually achieved acoustic attenuation and the settings of active components of the earplug, is stored in the database 10 for further use, which is particularly useful if a new pair of earplugs is to be produced for replacing the original one or if a modified version of the earplugs is to be produced due to changes in the configuration data (for example, if the worker is exposed to a different noise environment, if the hearing disability of the worker has changed, etc.).

Preferably, the collection of the configuration data, the ear shape data and the fitting procedure are carried out at the same location. This location preferably is a location of the company for which the user works so that the user does not have to go to a remote location in order to collect the user-specific data and to fit the product to the user. Alternatively, this location may be a transportation hub such as an airport or a central station of public transportation, for example, a central train station, a central subway station or a central tramway station. Also in this case there is no need for the user to go to a remote location for being provided with the hearing protection earplugs. As a further alternative, this location may be a mobile location, such a bus, a truck or a tent, which preferably is brought to the location where the user works or to a transportation hub or at least close to such a location in order to avoid a need of the user to go to a remote location only for the purpose of collecting data for the production of a customized hearing protection earplug.

The actual manufacturing steps, i.e. shell modelling, shell manufacturing and earplug assembly usually will be carried out at one or several locations remote from the data collection/fitting location. Preferably, the configuration data, ear shape data and fitting data collected from the user are transmitted via the internet or a similar network to the database 10 for further use in the manufacturing process.

In order to provide for a fast overall process, at least one of the actual manufacturing steps may be carried out in a time zone different from the time zone in which the data collection/fitting steps are carried out. For example, the shell modelling steps and the shell production steps could be carried out in different time zones for acceleration of the manufacturing process, since the therefore required working manpower could be made available for 24 hours per day.

In order to achieve a particularly convenient and fast overall process, the shell production steps and the earplug assembly steps could be carried out at the location where the data collection/fitting steps are carried out, since thereby the distance of the physical delivery of the product earplugs can be minimized. It is not necessary that the shell modelling steps are carried out a the same location; rather the shell modelling steps could be carried out at a remote location, for example, in a different time zone in order to accelerate the process, as mentioned above.

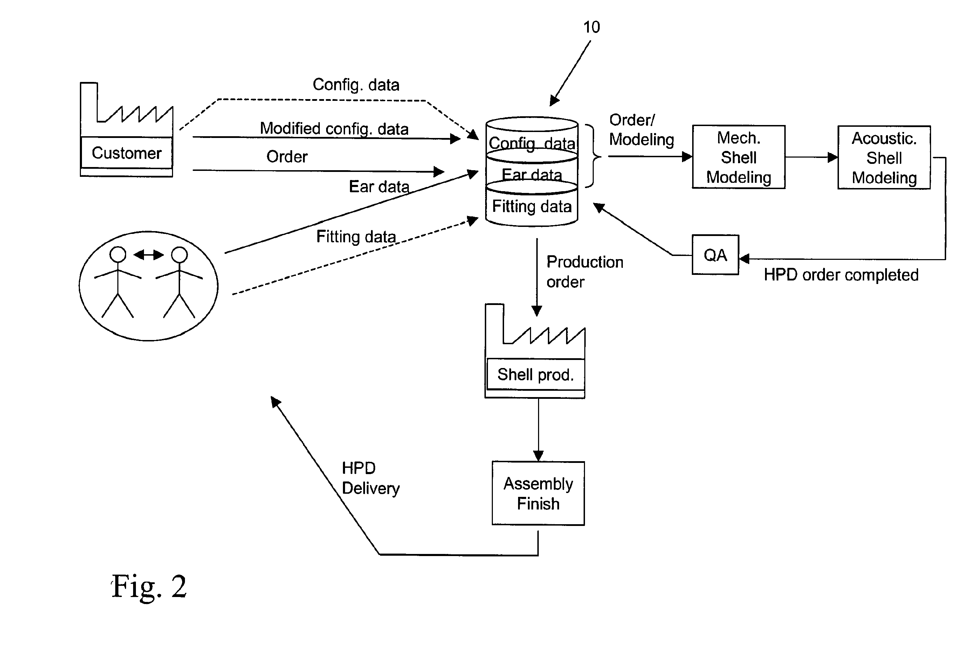

Fig. 2 shows a schematic example of a method for providing the user with a further, i.e. second, hearing protection earplug product after having provided the user already with an initial, i.e. first, hearing protection product, for example, according to the process shown in Fig. 1.

During production of the initial product earplug configuration data, ear shape data and fitting data specific to the user already have been collected and stored in the database 10 (see dashed lines in Fig. 2). In particular, the configuration data, ear shape data and fitting data may have been combined into a combined data set which is permanently stored in the database 10. Once a corresponding order has been received from the customer, i.e. the company for which the user works, a new hearing protection earplug product will be produced based on the stored configuration data, ear shape data and fitting data. By taking into account the fitting data, the new product earplugs may be optimized regarding the desired functionality expressed by the configuration data. For example, the new product earplugs may be delivered already with the optimized settings determined by the fitting procedure of the original product earplugs.

If a new shell identical to the original shell is acceptable and if the data representative of the modelled shell has been stored when producing the original product earplugs, the shell modelling steps may be omitted and the new shell may be manufactured based on such stored previous modelled shell data.

However, alternatively the customer may desire product earplugs which take into account modifications of the configuration data which may have occurred since the original product earplugs have been delivered, such as a different noise environment due to a change of the working place of the user, because the user now works in a different room or at a different machine, changes of the hearing disability of the user, changes of the communication needs of the user at his working place, etc. In this case, the order for new hearing protection earplugs will include modified configuration data taking into account such changes. In this case, a new shell will be modelled based on the original ear shape data and the modified configuration data provided by the customer; subsequently the new shell will be produced based on this new modelled shell. Thereby the hearing protection earplugs used by the worker can be adapted to changes in the audio needs of the worker in a particularly fast, convenient and efficient manner.

## Claims

1. A method for providing a user with a hearing protection earplug including a shell to be worn at least in part in the ear canal of the user, comprising
(1) collecting configuration data about audio needs of the user;
(2) collecting data of the inner shape of the outer ear and ear canal of the user by measuring the actual inner shape of the user's outer ear and ear canal;
(3) determining, based on said configuration data and said actual ear shape data, the desired shape of the shell;
(4) manufacturing said shell based on said determined desired shape by an additive layer-by-layer build-up process;
(5) producing a hearing protection earplug from said shell;
(6) fitting said hearing protection earplug to the user for verifying that said hearing protection earplug complies with the configuration data collected in step (1),
**characterized in that**
at least one of steps (1), (2), and (6) is carried out at a transportation hub.

2. The method of claim 1, wherein said transportation hub is an airport or a central station of public transportation such as a central train station, a central subway station or a central tramway station.

3. The method of one of claims 1 or 2, wherein at least steps (2) and (6) are carried out at said transportation hub.

4. The method of claim 3, wherein all of steps (1) to (6) are carried out at said transportation hub.

5. A method for providing a user with a hearing protection earplug including a shell to be worn at least in part in the ear canal of the user, comprising
(1) collecting configuration data about audio needs of the user;
(2) collecting data of the inner shape of the outer ear and ear canal of the user by measuring the actual inner shape of the user's outer ear and ear canal;
(3) determining, based on said configuration data and said actual ear shape data, the desired shape of the shell;
(4) manufacturing said shell based on said determined desired shape by an additive layer-by-layer build-up process;
(5) producing a hearing protection earplug from said shell;
(6) fitting said hearing protection earplug to the user for verifying that said hearing protection earplug complies with the configuration data collected in step (1),
**characterized in that**
at least one of steps (1), (2) and (6) is carried out in a mobile location.

6. The method of claim 5, wherein said mobile location is a bus, a truck or a tent.

7. The method of one of claims 5 or 6, wherein at least steps (2) and (6) are carried out in said mobile location.

8. The method of claim 7, wherein all of steps (2) to (6) are carried out in said mobile location.

9. The method of one of claims 5 to 8, wherein said mobile location is brought to a transportation hub or to a site at the company for which the user works.

10. A method for providing a user with a hearing protection earplug including a shell to be worn at least in part in the ear canal of the user, comprising
(1) collecting configuration data about audio needs of the user;
(2) collecting data of the inner shape of the outer ear and ear canal of the user by measuring the actual inner shape of the user's outer ear and ear canal;
(3) determining, based on said configuration data and said actual ear shape data, the desired shape of the shell;
(4) manufacturing said shell based on said determined desired shape by an additive layer-by-layer build-up process;
(5) producing a hearing protection earplug from said shell;
(6) fitting said hearing protection earplug to the user for verifying that said hearing protection earplug complies with the configuration data collected in step (1),
**characterized in that**
in step (6) data regarding the fitted hearing protection earplug, such as the mechanical acoustic attenuation achieved, is collected and said configuration data, said ear shape data and said fitting data are permanently stored and are used for manufacturing a second shell and a second earplug.

11. The method of claim 10, wherein subsequent to step (6) an order including modified configuration data for said second earplug is collected from the user or the company for which the user works, and wherein said second earplug is produced based on modified configuration data, said ear shape data, and said fitting data.

12. A method for providing a user with a hearing protection earplug including a shell to be worn at least in part in the ear canal of the user, comprising
(1) collecting configuration data about audio needs of the user;
(2) collecting data of the inner shape of the outer ear and ear canal of the user by measuring the actual inner shape of the user's outer ear and ear canal;
(3) determining, based on said configuration data and said actual ear shape data, the desired shape of the shell;
(4) manufacturing said shell based on said determined desired shape by an additive layer-by-layer build-up process;
(5) producing a hearing protection earplug from said shell;
(6) fitting said hearing protection earplug to the user for verifying that said hearing protection earplug complies with the configuration data collected in step (1),
**characterized in that**
at least one of steps (1), (2) and (6) is carried out at a site of the company for which the user works.

13. The method of claim 12, wherein at least steps (2) and (6) are carried out at a site of the company for which the user works.

14. The method of claim 13, wherein all of steps (1) to (6) are carried out at a site of the company for which the user works.

15. A method for providing a user with a hearing protection earplug including a shell to be worn at least in part in the ear canal of the user, comprising
(1) collecting configuration data about audio needs of the user;
(2) collecting data of the inner shape of the outer ear and ear canal of the user by measuring the actual inner shape of the user's outer ear and ear canal;
(3) determining, based on said configuration data and said actual ear shape data, the desired shape of the shell;
(4) manufacturing said shell based on said determined desired shape by an additive layer-by-layer build-up process;
(5) producing a hearing protection earplug from said shell;
(6) fitting said hearing protection earplug to the user for verifying that said hearing protection earplug complies with the configuration data collected in step (1),
**characterized in that**
at least one of steps (3) to (5) is carried out in a time zone different to the time zone in which steps (2) and (6) are carried out.

16. The method of claim 15, wherein steps (3) and (4) are carried out in different time zones.

17. The method of one of the preceding claims, wherein steps (4) and (5) are carried out at the same site.

18. The method of claim 17, wherein step (3) is carried out at a site different from the site at which steps (4) and (5) are carried out.

19. The method of one of the preceding claims, wherein said ear shape data is transferred via the internet to the site where step (3) is carried out.

20. The method of one of the preceding claims, wherein in step (2) the user is provided with a personal copy of said ear shape data for free personal use.

21. The method of one of the preceding claims, wherein step (2) is carried out according to a standardized process.

22. The method of one of the preceding claims, wherein step (6) further includes at least one of the following substeps: in-situ measuring the mechanical acoustic attenuation of the assembled earplug when worn by the user, providing the user with handling instructions; visible check of proper fit of the shell with the user's ear canal; and adjusting acoustical and mechanical properties of the earplug.

23. The method of one of the preceding claims, wherein in step (3) at least one functional component of said earplug is selected, based on said configuration data and said ear shape data, from a library of functional components.

24. The method of one of the preceding claims, wherein in step (3) data representative of said desired shape is permanently stored.

25. The method of one of the preceding claims, wherein in step (5) said additional functional components are assembled with said shell.

26. The method of one of the preceding claims, wherein in step (6) said additional functional components are adjusted if necessary.

27. The method of one of the preceding claims, wherein said configuration data includes data regarding the manner of use of the hearing protection earplug as intended by the user, the hearing disability of the user and/or the expected noise exposure in the environments in which the hearing protection earplug is intended to be worn.

28. The method of one of the preceding claims, wherein in step (2) said ear shape data is collected by taking an impression of the inner shape of the user's outer ear and ear canal and scanning the impression or by direct scanning of the inner shape of the user's outer ear and ear canal.

29. The method of one of the preceding claims, wherein said additive layer-by-layer build-up process includes layer-by-layer laser sintering of a powder material.

30. The method of one of the preceding claims, wherein said shell has an elasticity of from shore D85 to shore D65.
